(19) Europäisches Patentamt
European Patent Office
Office européen des brevets

(11) Veröffentlichungsnummer: **0 330 041 B1**

# (12) EUROPÄISCHE PATENTSCHRIFT

(45) Veröffentlichungstag der Patentschrift: **13.04.94**

(21) Anmeldenummer: **89102429.1**

(22) Anmeldetag: **13.02.89**

(51) Int. Cl.5: **C07D 417/12**, C07D 413/12, A61K 31/53, A01N 43/76, A01N 43/78, C07D 253/06, C07C 271/08

## (54) Substituierte 1,2,4-Triazindione, Verfahren zu ihrer Herstellung und ihre Verwendung.

(30) Priorität: **24.02.88 DE 3805660**

(43) Veröffentlichungstag der Anmeldung:
**30.08.89 Patentblatt 89/35**

(45) Bekanntmachung des Hinweises auf die Patenterteilung:
**13.04.94 Patentblatt 94/15**

(84) Benannte Vertragsstaaten:
**AT BE CH DE ES FR GB GR IT LI NL SE**

(56) Entgegenhaltungen:
**EP-A- 0 153 617**
**DE-A- 2 532 363**
**DE-A- 2 722 537**
**FR-A- 2 231 378**

(73) Patentinhaber: **BAYER AG**

**D-51368 Leverkusen(DE)**

(72) Erfinder: **Lindner, Werner, Dr.**
**Märchenstrasse 39**
**D-5000 Köln 80(DE)**
Erfinder: **Haberkorn, Axel, Prof. Dr.**
**Fuhlrottstrasse 99**
**D-5600 Wuppertal 1(DE)**

**EP 0 330 041 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue substituierte 1,2,4-Triazindione, Verfahren zu ihrer Herstellung, Zwischenprodukte zur Durchführung dieser Verfahren, sowie ihre Verwendung.

Die Verwendung von substituierten 1,2,4-Triazintrionen zur Bekämpfung von Coccidien ist bekannt (EP-OS 153 617). Auch die Verwendung von 2-Phenyl-1,2,4-triazin-3,5-dionen zur Bekämpfung von Coccidien ist bekannt (DE-A 2 722 537; FR-A 17 417 284; DE-A 2 532 363). Die Wirkung dieser Verbindungen befriedigt jedoch nicht in jedem Fall.

Die vorliegende Erfindung betrifft

1. Neue substituierte 1,2,4-Triazindione der allgemeinen Formel I

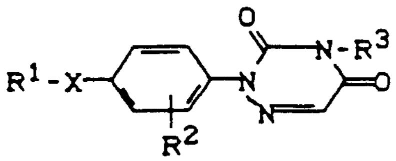

I

in welcher

- $R^1$ für über Kohlenstoff gebundene heteroaromatische Reste steht, die gegebenenfalls substituiert sind,
- X für O, S, SO, $SO_2$ steht,
- $R^2$ für einen oder mehrere, gleiche oder verschiedene Reste der Gruppe Wasserstoff, Halogen, Nitro, Alkyl, Alkoxy, Alkylthio, Halogenalkyl, Halogenalkoxy und Halogenalkylthio
- $R^3$ für Wasserstoff, gegebenenfalls substituiertes Alkyl, Alkenyl, Alkinyl, Aralkyl steht.

2. Verfahren zur Herstellung substituierte 1,2,4-Triazindione der allgemeinen Formel I

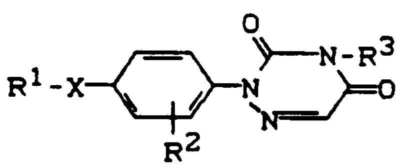

I

in welcher

- $R^1$ für über Kohlenstoff gebundene heteroaromatische Reste steht, die gegebenenfalls substituiert sind,
- X für O, S, SO, $SO_2$ steht,
- $R^2$ für Wasserstoff einen oder mehrere, gleiche oder verschiedene Reste der Gruppe Wasserstoff, Halogen, Nitro, Alkyl, Alkoxy, Alkylthio, Halogenalkyl, Halogenalkoxy und Halogenalkylthio
- $R^3$ für Wasserstoff, gegebenenfalls substituiertes Alkyl, Alkenyl, Alkinyl, Aralkyl steht,

dadurch gekennzeichnet, daß man

a) Verbindungen der Formel II

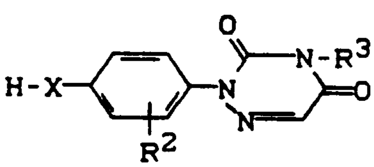

II

in welcher

- X für O oder S steht,
- $R^2$ $R^3$ die oben angegebene Bedeutung haben

mit Verbindungen der Formel III

$R^1$-A (III)

in welcher

$R^1$ die oben angegebene Bedeutung hat und

A für die Reste Halogen, O-$SO_2$-Alkyl, -O-$SO_2$-Halogenalkyl, -O-$SO_2$-Aryl, -S-Alkyl, -$SO_2$-Alkyl oder $SO_2$-Halogenalkyl steht

umsetzt, oder

b) daß man zur Herstellung von Verbindungen der Formel I in welcher $R^3$ nicht für Wasserstoff steht Verbindungen der Formel Ia

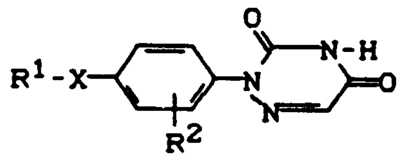

Ia

in welcher

$R^1$, $R^2$, X die oben angegebene Bedeutung haben

mit Verbindungen der Formel IV

$R^3$-B (IV)

in welcher

$R^3$ für gegebenenfalls substituiertes Alkyl, Alkenyl, Alkinyl, Aralkyl steht und

B für Halogen, -O-$SO_2$-Alkyl, -O-$SO_2$-Aryl, -O-$SO_2$-Halogenalkyl steht

umsetzt, oder

c) daß man zur Herstellung von Verbindungen der Formel I in welcher X für -SO- oder -$SO_2$-steht, Verbindungen der Formel I in welcher X für S steht mit einem Oxidationsmittel umsetzt.

3. Neue Verbindungen der Formel II

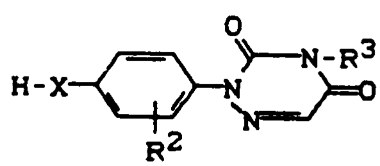

II

in welcher

X für O oder S steht,

$R^2$ für einen oder mehrere, gleiche oder verschiedene Reste an der Gruppe Halogen, Nitro, Alkyl, Alkoxy, Alkylthio, Halogenalkyl, Halogenalkoxy und Halogenalkylthio steht und für den Fall, daß X für S steht, zusätzlich für Wasserstoff steht,

$R^3$ für Wasserstoff, Alkyl, Alkenyl, Alkinyl, Aralkyl steht.

4. Verfahren zur Herstellung der neuen Verbindungen der Formel II gemäß (3), dadurch gekennzeichnet, daß man Verbindungen der Formel V

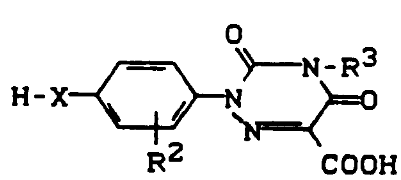

V

in welcher

X, $R^2$, $R^3$ die in (3) angegebene Bedeutung haben,

durch Erhitzen decarboxyliert.

5. Neue Verbindungen der Formel V

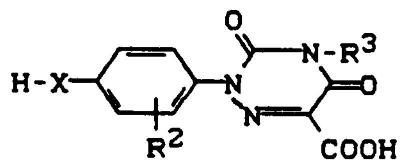

V

in welcher

X für O oder S steht,

$R^2$ für einen oder mehrere, gleiche oder verschiedene Reste aus der Gruppe Halogen, Nitro, Alkyl, Alkoxy, Alkylthio, Halogenalkyl, Halogenalkoxy und Halogenalkylthio steht und für den Fall, daß X für S steht, zusätzlich für Wasserstoff steht,

$R^3$ für Wasserstoff, Alkyl, Alkenyl, Alkinyl, Aralkyl steht.

6. Verfahren zur Herstellung der neuen Verbindungen der Formel V gemäß (5), dadurch gekennzeichnet, daß man Verbindungen der Formel VI

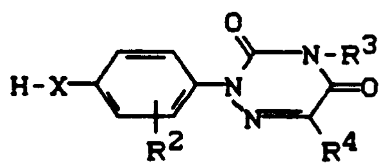

VI

in welcher

X, $R^2$, $R^3$ die in (3) angegebene Bedeutung haben,

$R^4$ für die Reste -CN,

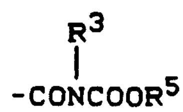

steht,

$R^5$ für gegebenenfalls substituiertes Alkyl oder Aryl steht

in Gegenwart wäßriger Säuren erhitzt.

7. Neue Verbindungen der Formel VI

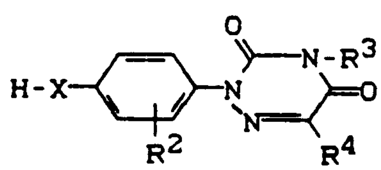

VI

in welcher

X, $R^2$, $R^3$, $R^4$ die in (6) angegebene Bedeutung haben.

8. Verfahren zur Herstellung der neuen Verbindungen der Formel VI gemäß (7) dadurch gekennzeichnet, daß man Verbindungen der Formel VII

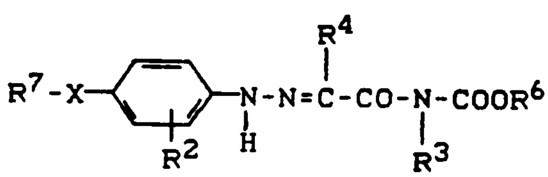

VII

in welcher

X, $R^2$, $R^3$, $R^4$ die in (7) angegebene Bedeutung haben und

$R^6$ für Alkyl oder gegebenenfalls substituiertes Aryl steht,

$R^7$ für Wasserstoff oder gegebenenfalls substituiertes

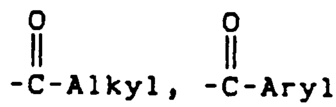

steht,

in Gegenwart von Basen erhitzt.

9. Neue Verbindungen der Formel VII

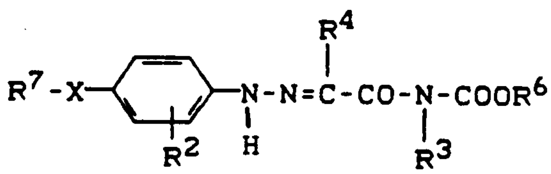

VII

in welcher

X, $R^2$, $R^3$, $R^4$, $R^6$ und $R^7$ die in (8) angegebene Bedeutung haben und für den Fall, daß $R^7$ für H oder X für S steht, $R^2$ zusätzlich für Wasserstoff stehen kann.

10. Verfahren zur Herstellung der neuen Verbindungen der Formel VII gemäß (9) dadurch gekennzeichnet, daß man Verbindungen der Formel VIII

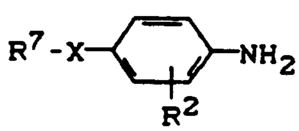

VIII

in welcher

X und $R^2$, $R^7$ die in (9) angegebene Bedeutung haben,

zunächst mit Alkalinitrit in Gegenwart von wäßrigen Mineralsäuren diazotiert und anschließend mit Verbindungen der Formel IX

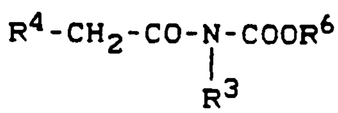

IX

in welcher

$R^3$, $R^4$ und $R^6$ die in (9) angegebene Bedeutung besitzen

umsetzt.

Die Verbindungen der Formel I sowie ihre Salze mit Säuren oder Basen sind hervorragend zur Bekämpfung parasitischer Protozoen geeignet.

Bevorzugte Verbindungen der Formel I sind Verbindungen in denen

$R^1$ für gegebenenfalls durch Halogen, Alkyl, Cyano, Nitro, o-Alkyl, s-Alkyl, Halogenalkyl, substituiertes Thiazolyl, Oxazolyl, Benzthiazolyl oder Benzoxazolyl steht.

X für O, S steht

$R^2$ für Halogen oder $C_{1-6}$-Alkyl steht

$R^3$ für Wasserstoff oder $C_1$-$C_4$-Alkyl, insbesondere Methyl, steht.

Besonders bevorzugt sind Verbindungen der Formel I in welcher

X für O steht,

$R^1$ für Thiazolyl, Benzthiazolyl oder Benzoxazolyl die gegebenenfalls durch $C_{1-4}$-Alkyl, insbesondere Methyl, $C_{1-4}$-Halogenalkyl insbesondere Trifluormethyl, Halogen, insbesondere Chlor, Brom, Fluor, Nitro, CN, Amino, $C_{1-4}$-Alkylamino, $C_{1-4}$-Halogenalkylamino,Acylamino, $C_{1-4}$-Alkoxy insbesondere Methoxy, $C_{1-4}$-Halogenalkoxy insbesondere Trifluormethoxy, $C_{1-4}$-Alkylthio insbesondere Methylthio, $C_{1-4}$-Halogenalkylthio insbesondere Trifluormethylthio, $C_{1-4}$-Alkylsulfonyl, insbesondere Methylsulfonyl und $C_{1-4}$-Halogenalkylsulfonyl, insbesondere Trifluormethylsulfonyl substituiert sind, steht

$R^2$ für einen oder mehrere Reste der Gruppe Wasserstoff oder Halogen insbesondere Chlor, Brom, $C_{1-4}$-Alkyl insbesondere Methyl steht,

$R^3$ für Wasserstoff steht.

Ganz besonders bevorzugt sind Verbindungen der Formel I in welcher

X für O steht,

$R^1$ für gegebenenfalls durch Chlor oder Methyl oder Trifluormethyl substituiertes Thiazolyl oder Benzthiazolyl steht,

$R^2$ für einen oder mehrere Reste der Gruppe Wasserstoff, Methyl oder Chlor steht,

$R^3$ für Wasserstoff steht.

Als Einzelverbindungen seien genannt:

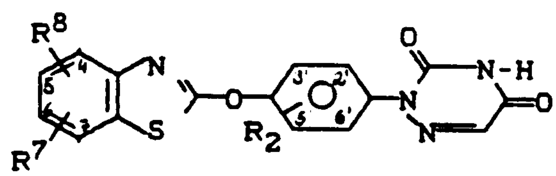

| $R_2$ | $R^7$ | $R^8$ |
|---|---|---|
| 3-$CH_3$ | 6-Cl | H |
| 3-$CH_3$ | 6-$CF_3$ | H |
| 3-$CH_3$ | 5-Cl | 6-Cl |
| 3,5-Cl | 6-Cl | H |
| 3,5-Cl | 6-$CF_3$ | H |
| 3,5-Cl | 5-Cl | 6-Cl |

Weiterhin seien die folgenden Verbindungen genannt:

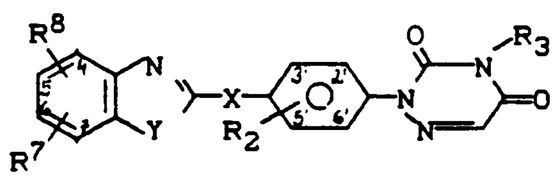

| Y | $R_2$ | X = O | $R_3$ | $R_7$ | $R_8$ |
|---|---|---|---|---|---|
| S | H | | H | H | H |
| S | H | | H | 6-Cl | H |
| S | H | | H | 6-Br | H |
| S | H | | H | 6-F | H |
| S | H | | H | 6-$CH_3$ | H |
| S | H | | H | 6-$OCH_3$ | H |
| S | H | | H | 6-$NO_2$ | H |
| S | H | | H | 6-CN | H |
| S | H | | H | 6-$CF_3$ | H |
| S | H | | H | 6-$SCF_3$ | H |
| S | H | | H | 6-$OCF_3$ | H |
| S | H | | H | 5-Cl | 6-Cl |
| S | 3'-$CH_3$ | | H | H | H |
| S | 3'-$CH_3$ | | H | 6-Br | H |
| S | 3'-$CH_3$ | | H | 6-F | H |

| Y | $R_2$ | X = O | $R_3$ | $R_7$ | $R_8$ |
|---|---|---|---|---|---|
| S | 3'-$CH_3$ | | H | 6-$CH_3$ | H |
| S | 3-$CH_3$ | | H | 6-$OCH_3$ | H |
| S | 3-$CH_3$ | | H | 6-$NO_2$ | H |
| S | 3-$CH_3$ | | H | 6-CN | H |
| S | 3-$CH_3$ | | H | 6-$SCF_3$ | H |
| S | 3-Cl | | H | H | H |
| S | 3-Cl | | H | 6-Cl | H |
| S | 3-Cl | | H | 6-Br | H |
| S | 3-Cl | | H | 6-F | H |
| S | 3'-Cl | | H | 6-$CH_3$ | H |
| S | 3'-Cl | | H | 6-$OCH_3$ | H |
| S | 3'-Cl | | H | 6-$NO_2$ | H |
| S | 3'-Cl | | H | 6-CN | H |
| S | 3'-Cl | | H | 6-$CF_3$ | H |
| S | 3'-Cl | | H | 6-$SCF_3$ | H |
| S | 3'-Cl | | H | 6-$OCF_3$ | H |
| S | 3'-Cl | | H | 5-Cl | 6-Cl |
| S | 3',5'-Cl | | H | H | H |
| S | 3',5'-Cl | | H | 6-Br | H |
| S | 3',5'-Cl | | H | 6-$CH_3$ | H |
| S | 3',5'-Cl | | H | 6-$OCH_3$ | H |
| S | 3',5'-Cl | | H | 6-$NO_2$ | H |
| S | 3',5'-Cl | | H | 6-CN | H |
| S | 3',5'-Cl | | H | 6-$SCF_3$ | H |
| S | 3',5'-Cl | | H | 6-$OCF_3$ | H |
| S | 3'-Cl, 5'-$CH_3$ | | H | H | H |
| S | 3'-Cl, 5'-$CH_3$ | | H | 6-Cl | H |
| S | 3'-Cl, 5'-$CH_3$ | | H | 6-Br | H |
| S | 3'-Cl, 5'-$CH_3$ | | H | 6-F | H |
| S | 3'-Cl, 5'-$CH_3$ | | H | 6-$OCH_3$ | H |

| Y | $R_2$ X = O | $R_3$ | $R_7$ | $R_8$ |
|---|---|---|---|---|
| S | 3'-Cl, 5'-$CH_3$ | H | 6-CN | H |
| S | 3'-Cl, 5'-$CH_3$ | H | 6-CN | H |
| S | 3'-Cl, 5'-$CH_3$ | H | 6-$CF_3$ | H |
| S | 3'-Cl, 5'-$CH_3$ | H | 6-$SCF_3$ | H |
| S | 3'-Cl, 5'-$CH_3$ | H | 5-Cl | 6-Cl |
| S | 3'-$CH_3$, 5'-$CH_3$ | H | 6-Cl | H |
| S | 3'-$CH_3$, 5'-$CH_3$ | H | 5-Cl | 6-Cl |
| S | 3'-$CH_3$, 5-$CH_3$ | H | 5-Cl | H |
| S | 3'-Cl | H | 5-Cl | H |
| S | 3'-$CH_3$ | H | 5-Cl | H |
| S | 3'-Cl, 5'-$CH_3$ | H | 5-Cl | H |
| S | 3'-Cl, 5'-Cl | H | 5-Cl | H |
| S | 3'-Br | H | 6-Cl | H |
| S | 3'-Br, 5'-Br | H | 6-Cl | H |
| S | 3'-$CF_3$ | H | 6-Cl | H |
| S | 3'-$CF_3$, 5'-Cl | H | 6-Cl | H |
| O | 3'-Cl, 5'-Cl | H | 6-Cl | H |
| O | 3'-$CH_3$ | H | 6-Cl | H |
| S | 3'-Cl, 5'-Cl | $CH_3$ | 6-CL | H |
| S | 3'-$CH_3$ | $-C_2H_5$ | 5-Cl | 6-Cl |

| Y | X | $R_2$ | $R_3$ | $R_7$ | $R_8$ |
|---|---|---|---|---|---|
| S | S | H | H | 6-Cl | H |
| S | S | H | H | H | H |
| O | S | H | H | H | H |
| O | SO | H | H | H | H |
| O | $SO_2$ | H | H | H | H |
| O | S | 3,5-$Cl_2$ | H | 6-Cl | H |
| O | S | 3,5-$Cl_2$ | H | H | H |

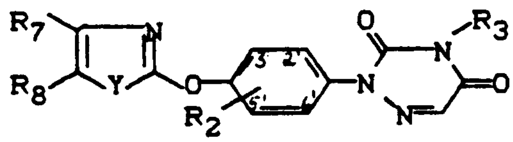

| Y | $R_2$ | $R_3$ | $R_7$ | $R_8$ |
|---|---|---|---|---|
| S | H | H | H | H |
| S | H | H | Cl | H |
| S | H | H | Cl | Cl |
| S | H | H | Cl | $CF_3$ |
| S | H | H | Cl | $CH_3$ |
| S | 3'-Cl | H | H | H |
| S | 3'-Cl | H | Cl | H |
| S | 3'-Cl | H | Cl | Cl |
| S | 3'-Cl | H | Cl | $CF_3$ |
| S | 3'-$CH_3$ | H | Cl | H |
| S | 3'-$CH_3$ | H | Cl | Cl |
| S | 3'-$CH_3$ | H | Cl | $CF_3$ |
| S | 3'-Cl, 5'-Cl | H | Cl | H |
| S | 3'-Cl, 5'-Cl | H | Cl | Cl |
| S | 3'-Cl, 5'-Cl | H | Cl | $CF_3$ |
| O | 3'-Cl, 5'-Cl | H | H | H |
| O | 3'-$CH_3$ | H | H | H |
| O | 3'-Cl, 5'-Cl | H | Cl | H |
| O | 3'-$CH_3$ | H | Cl | H |
| S | 3'-Cl, 5'-Cl | $CH_3$ | Cl | Cl |
| S | 3'-$CH_3$ | $C_2H_5$ | Cl | Cl |

Setzt man bei dem Verfahren 2 als Verbindung II 2-(3,5-Dichloro-4-hydroxyphenyl)-1,2,4-triazin-3,5-(2H,4H)dion und als Verbindung der Formel III 2,6-Dichlorobenzthiazol ein läßt sich das Verfahren durch folgendes Formelschema beschreiben.

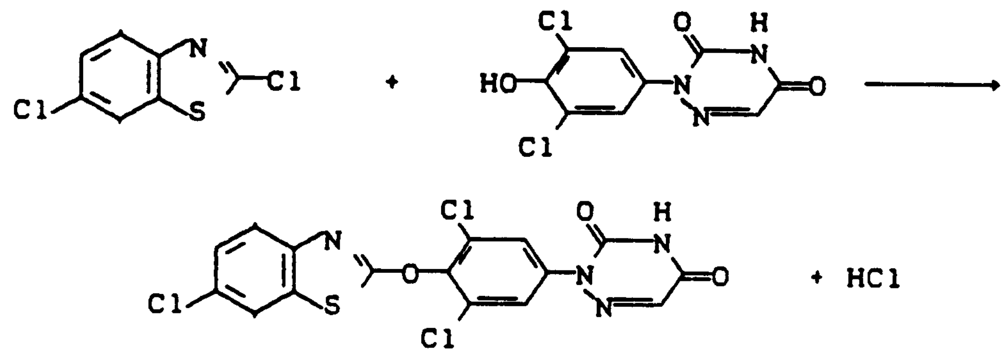


Verbindungen der Formel II in welcher $R^2$ und $R^3$ für Wasserstoff stehen sind bekannt (J. Slouka, Acta Unio Palacki Olomuk. Fac. Rerum. Nat. 1984 (Chem 23), 39-45; C.A. 102 203946c).

Verbindungen der Formel II in welcher $R^2$ für andere Reste als Wasserstoff steht sind neu.

Bevorzugt seien Verbindungen der Formel II genannt in denen $R^2$ und $R^3$ die bei den Verbindungen der Formel I genannten bevorzugten Bedeutungen haben.

Im einzelnen seien folgende neue Verbindungen der Formel II genannt.

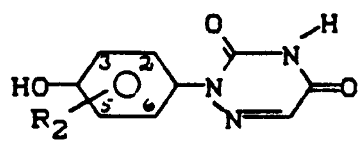

| $R^2$ |
|---|
| 3-Cl |
| 3-$CH_3$ |
| 3,5-Cl |
| 3-$CH_3$, 5-$CH_3$ |
| 3,5-$CH_3$ |

Die substituierten Heterocyclen der Formel III sind bekannt oder lassen sich analog zu bekannten Verfahren herstellen (Beilstein Bd. 27; Katrizky und Rees, Comprehensive Het. Chem. Col. 6 1984).

Sie besitzen die weiter oben bei den Verbindungen der Formel I angegebenen bevorzugten Bedeutungen. Im einzelnen seien die folgenden Verbindungen der Formel III genannt.

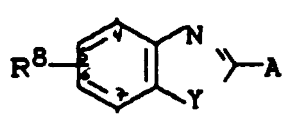

| Y | $R^8$ | A |
|---|---|---|
| S | 6-Cl | Cl |
| S | 5,6-Cl | Cl |
| O | 6-Cl | Cl |
| O | 5,6-Cl | Cl |
| S | 6-Cl | $-SO_2CH_3$ |
| S | 5,6-Cl | $-SO_2CH_3$ |
| O | 6-Cl | $-SO_2CH_3$ |

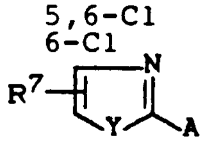

| Y | $R^7$ | A |
|---|---|---|
| S | 4-Cl | Cl |
| S | 4,5-Cl | Cl |
| O | 4-Cl | Cl |
| O | 4,5-Cl | Cl |

Die Reaktion wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl- ethyl-, Methyl-isopropyl- und Methyl-isobutyl-keton, ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuertriamid.

Die Reaktion wird in Gegenwart von anorganischen oder organischen Säureakzeptoren durchgeführt.

Als solche seien genannt z.B.:
Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Pyridin, 1,5-Diazabiccylo-[4,3,0]-non-5-en (DBN), 1,8-Diazabicyclo-[5,4,0]-undec-7-en (DBU) und 1,4-Diazabicyclo-[2,2,2]-octan (DABCO).

Die Umsetzung erfolgt bei Temperaturen zwischen 50 und 200 °C vorzugsweise zwischen 80 und 160 °C bei Normaldruck oder erhöhtem Druck. Es wird bevorzugt bei Normaldruck gearbeitet.

Das Verfahren wird durchgeführt indem äquimolare Mengen der Verbindungen der Formel II und III in einem der angegebenen Verdünnungsmittel zusammengegeben und erhitzt werden. Nach vollendeter Umsetzung wird das Reaktionsgemisch mit verdünnter anorganischer Säure (z.B. Salzsäure) angesäuert und der entstehende Niederschlag abfiltriert, gewaschen und getrocknet.

Setzt man bei dem Verfahren 2b zur Herstellung der Verbindungen der Formel I in welcher $R^3$ nicht für Wasserstoff steht, als Verbindung der Formel Ia 2-[4-[2'-Benzthiazolyloxy]phenyl]1,2,4-triazin-3,5-(2H,4H)-dion und als Verbindung der Formel IV Methyljodid ein läßt sich das Verfahren durch folgendes Schema beschreiben.

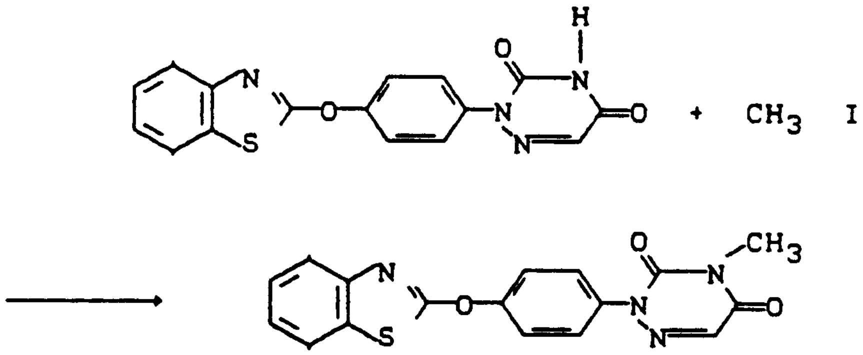


Die Verbindungen der Formel Ia sind neu und werden wie bei Verfahren 2a beschrieben dargestellt.

Die Verbindungen der Formel IV sind bekannt oder lassen sich nach bekannten Methoden darstellen. Besonders genannt sei Methyliodid, Ethylbromid.

Das Verfahren wird durchgeführt indem man eine Verbindung der Formel Ia in Gegenwart einer Base und eines Verdünnungsmittels mit Verbindungen der Formel IV umsetzt. Als Verdünnungsmittel können alle inerten organischen Lösungsmittel eingesetzt werden die auch zur Durchführung von Verfahren Ia dienen.

Das Verfahren wird in Gegenwart von Basen durchgeführt. Als bevorzugte Basen seien genannt die Alkalihydroxide wie Natriumhydroxid, Alkalialkoholate wie Natriummethylat oder Kaliumbutanolat, Metallhydride wie Natriumhydrid oder organische Basen wie 1,8-Diazabicyclo[5,40]-undec-7-en (DBU).

Das Verfahren wird durchgeführt bei Normaldruck und Temperaturen zwischen 20° und 140°C.

Die Reaktion wird durchgeführt indem man äquimolare Mengen der Verbindung der Formel Ia und Base zusammengibt, dieses Gemisch mit einer äquimolaren Menge der Verbindung der Formel IV versetzt und auf die Reaktionstemperatur erhitzt.

Setzt man bei dem Verfahren 2c) zur Herstellung der Verbindungen der Formel I mit X = SO oder $SO_2$ als Verbindung der Formel I 2-[4-[(2'-Benzoxazolylthio)phenyl]1,2,4-triazin-3,5-(2H,4H)-dion ein läßt sich das Verfahren durch folgendes Schema beschreiben.

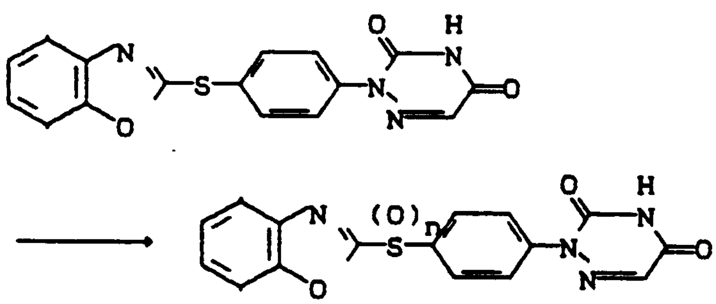

Das Verfahren wird durchgeführt indem man eine Verbindung der Formel I mit X = S in Gegenwart eines Verdünnungsmittels mit einem Oxidationsmittel behandelt. Als Oxidationsmittel finden vorzugsweise Verwendung: Wasserstoffperoxid und andere anorganische Peroxide wie Natriumperoxid, organische Peroxosäuren wie z.B. m-Chlorperbenzoesäure, Iodsauerstoffverbindungen wie z.B. Natriummetaperiodat.

Als Verdünnungsmittel können vorzugsweise eingesetzt werden: Alkohole wie z.B. Methanol, organische Säuren wie z.B. Essigsäure, weiterhin finden Ketone wie Aceton, halogenierte Kohlenwasserstoffe wie Dichlormethan oder Säureanhydride wie Acetanhydrid Verwendung. Die Oxidation erfolgt bei Temperaturen zwischen 0°C und 120°C. Es wird bevorzugt unter Normaldruck gearbeitet.

Die Menge des Oxidationsmittels kann zwischen einfach molar und 10fach molar variiert werden. Die Reaktion wird durchgeführt indem man die Verbindungen der Formel I mit X = S zusammen mit einem der angegebenen Oxidationsmittel in einem der oben angegebenen Verdünnungsmittel mehrere Stunden bei der angegebenen Reaktionstemperatur rührt.

Wird bei dem Verfahren 4 zur Herstellung der Verbindungen der Formel II als Verbindung der Formel V 2-(3-Methyl-4-hydroxyphenyl)-1,2,4-triazin-2,5(2H,4H)dion-6-carbonsäure eingesetzt, läßt sich das Verfahren durch folgendes Schema beschreiben:

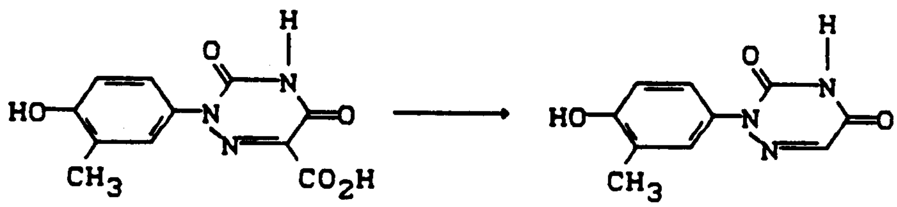

Die Verbindung der Formel V in welcher X für O und $R^2$ und $R^3$ für Wasserstoff stehen ist bekannt (J. Slouka, C.A. 102, 203946 k).

Die Verbindungen der Formel V in welcher $R^2$ nicht für Wasserstoff steht sind neu.

Die neuen Verbindungen der Formel V lassen sich analog zu bekannten Verfahren (DE-OS 2 358 851; J. Slouka, Mh. Chem. 96, 124 (1965)) herstellen.

Als Einzelverbindungen der Formel V seien genannt:

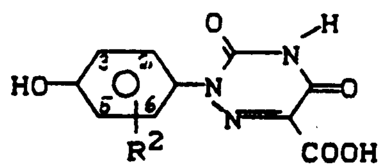

| $R_2$ |
|---|
| 3-Cl |
| 3-$CH_3$ |
| 3,5-Cl |
| 3-$CH_3$, 5-Cl |
| 3,5-$CH_3$ |

Die Decarboxylierung wird gegebenenfalls in Gegenwart von inerten organischen Verdünnungsmitteln durchgeführt. Hierzu gehören aliphatische und aromatische, gegebenenfalls halogenierte Kohlenwasserstoffe wie Nonan, Decan, Dodecan, Xylole, Ether wie Ethylenglykolmonobutylether, Diethylenglykoldibutylether.

Darüberhinaus kann die Reaktion in Gegenwart von Mercaptogruppenhaltigen Carbonsäuren wie z.B. Mercaptoessigsäure oder Thiosalicylsäure durchgeführt werden.

Die Umsetzung erfolgt bei Temperaturen zwischen 150 und 300 °C, gegebenenfalls in Gegenwart von mercaptogruppenhaltigen Carbonsäuren wie z.B. Mercaptoessigsäure vorzugsweise zwischen 160 und 250 °C, insbesondere zwischen 180 und 210 °C.

Es wird bei Normaldruck gearbeitet. Die Verbindungen der Formeln V werden in Substanz oder im jeweiligen Verdünnungsmittel, gelöst oder suspendiert, erhitzt.

Wird im Verfahren 6 zur Herstellung der Verbindungen der Formel V als Verbindung der Formel VI 2-(3-Methyl-4-hydroxyphenyl)-6-cyano-1,2,4-triazin 3,5(2H,4H)dion eingesetzt läßt sich das Verfahren durch folgendes Formelschema beschreiben.

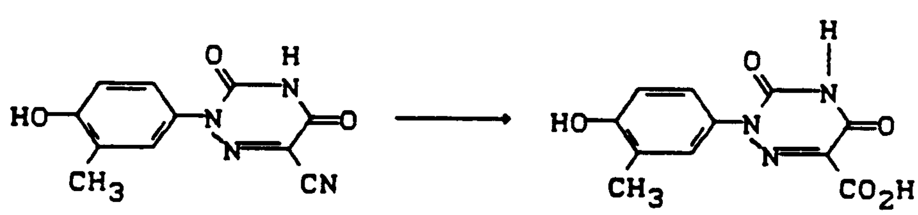

Die Verbindung der Formel VI in welcher X für O steht und $R^4$ für CN steht ist bekannt (J. Slouka, C.A. 102, 203946 K).

Die Verbindungen der Formel VI in welcher $R^2 \neq$ Wasserstoff ist, sind neu.

Die neuen Verbindungen der Formel VI lassen sich analog zu bekannten Verfahren (J. Slouka, Mh. Chem. 96, 134 (1965); DOS 2 358 851) herstellen.

Als Einzelverbindungen der Formel V seien genannt:

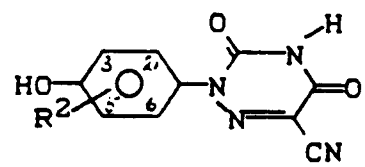

| $R_2$ |
|---|
| 3-Cl |
| 3-$CH_3$ |
| 3,5-Cl |
| 3-$CH_3$, 5-Cl |
| 3,5-$CH_3$ |

Die Hydrolyse wird unter sauren Bedingungen durchgeführt. Als Säuren kommen Mineralsäuren zum Einsatz wie z.B. Salzsäure, Bromwasserstoffsäure, Schwefelsäure und Gemische aus Mineralsäuren und organischen Säuren wie z.B. Essigsäure oder Propionsäure.

Die Umsetzung erfolgt bei Temperaturen zwischen 80 und 120 °C. Es wird unter Normaldruck gearbeitet.

Die Verbindungen der Formel VI werden im 10-30 fachen Volumen der Säure oder des Säuregemisches gelöst und bis zur abgeschlossenen Hydrolyse erhitzt.

Wird im Verfahren 8 zur Herstellung der Verbindungen der Formel VI als Verbindung der Formel VII Ethyl-N-[[[Cyano(3-methyl-4-hydroxyphenyl)-hydrazinyliden]methyl]carbonyl]-carbamat eingesetzt läßt sich das Verfahren durch folgendes Schema beschreiben:

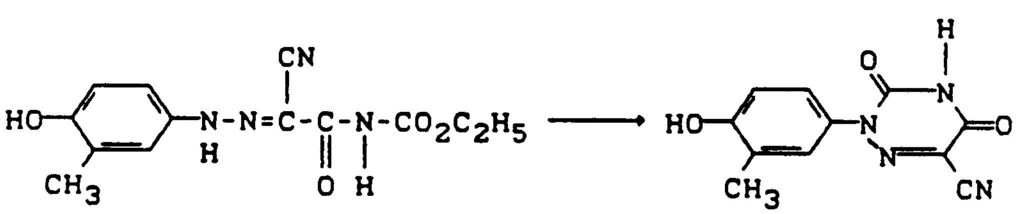

Die Verbindungen der Formel VII sind neu. Sie lassen sich analog zu bekannten Verfahren darstellen (J. Slouka, Mh. Chem. 94, 258 (1963)).

Als Einzelverbindungen der Formel VII seien genannt:

| $R^2$ | $R^4$ | $R^6$ |
|---|---|---|
| 3-Cl | -CN | $-C_2H_5$ |
| 3-$CH_3$ | -CN | $-C_2H_5$ |
| 3,5-Cl | -CN | $-C_2H_5$ |
| 3,5-$CH_3$ | -CN | $-C_2H_5$ |

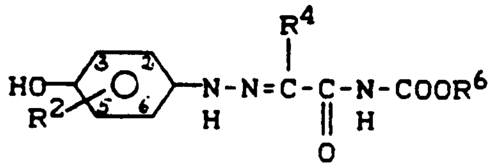

Das Verfahren wird durchgeführt indem man eine Verbindung der Formel VII erhitzt, gegebenenfalls in Gegenwart eines Lösungsmittels und einer Base.

Als Lösungsmittel und Basen finden die bei der Herstellung der Verbindungen I aufgeführten Lösungsmittel Verwendung. Als weitere besonders bevorzugte organische Lösungsmittel werden Alkohole wie z.B. Ethanol oder organische Säuren wie z.B. Eisessig eingesetzt.

Besonders bevorzugte Basen sind die Hydroxide und Acetate der Alkali- oder Erdalkalimetalle wie z.B. NaOH oder Natrium und Kaliumacetat.

Die Umsetzung erfolgt unter Normaldruck bei Temperaturen zwischen 70 und 150 °C, vorzugsweise zwischen 70 und 100 °C.

Die verwendete Base wird in 10-80 %igem molarem Überschuß eingesetzt. Das Reaktionsgemisch wird nach abgeschlossener Cyclisierung vorzugsweise mit einer verdünnten Mineralsäure wie z.B. Salzsäure angesäuert und das als Feststoff anfallende Produkt abfiltriert.

Wird im Verfahren 10 zur Herstellung der Verbindungen der Formel VII als Verbindung der Formel VIII 3-Methyl-4-hydroxanilin eingesetzt und als Verbindung der Formel IX Ethyl-cyanacetylurethan läßt sich das Verfahren durch folgendes Schema beschreiben:

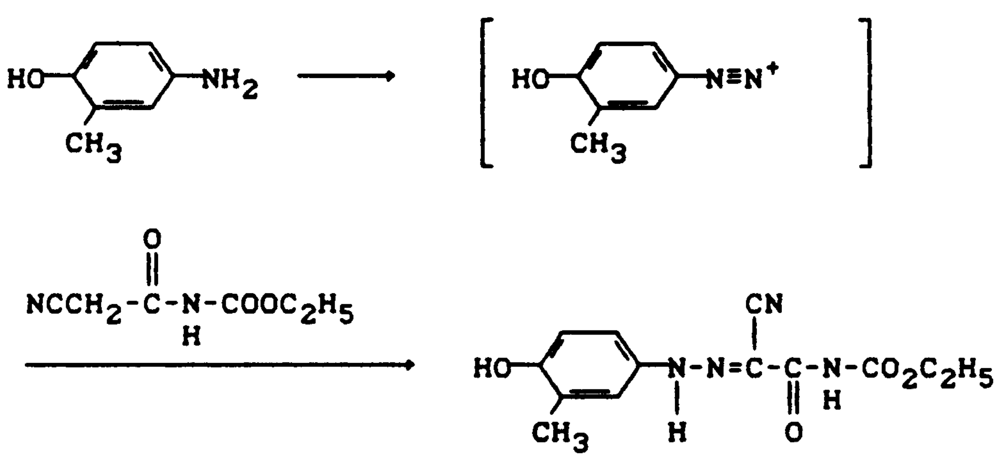

Die Verbindungen der Formel VIII und IX sind bekannt oder lassen sich analog zu bekannten Verfahren herstellen.

Das Verfahren wird durchgeführt indem man ein Anilin der Formel VIII mit $NaNO_2$ und konz. Mineralsäure wie z.B. HCl, gegebenenfalls in Gegenwart eines Verdünnungsmittels, umsetzt.

Als Verdünnungsmittel dienen mit Wasser mischbare Verdünnungsmittel wie Alkohole, z.B. Methanol, Ethanol, organische Säuren wie z.B. Eisessig, Ameisensäure, Glycolether wie Monomethylglycolether, Nitrile wie Acetonitril, Dimethylsulfoxid.

Das so erzeugte Diazoniumsalz wird in situ mit einer Verbindung der Formel IX wie z.B. Malonyldiurethan oder Cyanacetylurethan in Gegenwart einer Base umgesetzt. Als Basen finden Verwendung Hydroxide und Carbonate der Alkali und Erdalkalimetalle sowie Acetate von Natrium, Kalium und Ammonium.

Weiterhin können organische Basen wie Pyridin oder Triethylamin verwendet werden.

Die Diazotierung wird bei Normaldruck und bei Temperaturen zwischen 0 °C und 10 °C durchgeführt. Die Zugabe der Verbindungen der Formel IX erfolgt bei 5° bis 20 °C. Anilin und Nitrit werden in äquimolaren Mengen in einem Überschuß Säure vorzugsweise der 2-3 fachen molaren Menge umgesetzt. Die CH acide Verbindung wird in 0-30 %igem molaren Überschuß vorzugsweise 10 %igen Überschuß zugesetzt. Die Base wird im 1,5-2,5-fachen molaren Überschuß zugesetzt.

Das Kupplungsprodukt aus Diazoniumsalz und CH-acider Verbindung ist im Reaktionsmedium unlöslich und kann als Feststoff isoliert werden.

Das Verfahren kann auch so geführt werden, daß ohne Isolierung der Verbindungen der Formel VII direkt Verbindungen der Formel VI entstehen. Dazu wird die Diazotierung der Aniline der Formel VIII und die Umsetzung mit den Urethanen der Formel IX in einem für die Cyclisierung geeigneten Verdünnungsmittel durchgeführt. Das Reaktionsgemisch wird nach erfolgter Diazotierung und Kupplung erwärmt und dann das Triazindion der Formel VI isoliert.

Als Verdünnungsmittel seien genannt: Alkohole wie Methanol, Ethanol.

Zur Cyclisierung wird das Reaktionsgemisch auf ca. 80 bis 120 °C, bevorzugt ca. 80 bis 100 °C, erwärmt.

Die Aufarbeitung erfolgt wie weiter oben bei der Herstellung der Verbindungen der Formel VI angegeben.

Die Wirkstoffe eignen sich bei günstiger Warmblütertoxizität zur Bekämpfung von parasitischen Protozoen die und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den parasitischen Protozoen zählen:

Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. dabliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I.rivolta, I. spec., I. suis, Cystisospora spec., Cryptosporidium spec. wie Toxoplasmadidae z.B. Toxoplasma gondii, wie Sarcocystidae z.B. Sarcocystis bovicanis, S. bovihominis, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Darüber hinaus sind die erfindungsgemäßen Verbindungen wirksam gegenüber verschiedenen zu den Helminthen (Würmern) zählenden Fischparasiten vor allem Saugwürmern (Trematoda, Monogenea) wie z.B. Gyrodactylus spec., Dactylogyrus spec., Pseudodactylogyrus spec., Diplozoon spec.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär,

Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester,

Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$,Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-$\beta$-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft, erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Beispielhaft sei der Einsatz bei der Coccidiose genannt:
Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesodnere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Beispiel A

Coccidiose bei Hühnern

9 bis 11 Tage alte Küken wurden mit 40000 sporulierten Oozysten von stark virulenten Stämmen von Eiveria acervulina, E. maxima und E. tenella, den Krankheiteserregern der intestinalen Coccidiose infiziert.

3 Tage vor der Infektion und 8 Tage nach der Infektion (Ende des Versuchs) wurde Wirkstoff in der angegebenen Konzentration im Futter der Tiere eingemischt verabreicht.

Die Zahl der Oozysten im Kot wurde mit Hilfe der McMaster-Kammer bestimmt (siehe Engelbrecht und Mitarbeiter "Parasitologische Arbeitsmehoden in Medizin und Veterinärmedizin", S. 172, Akademie-Verlag, Berlin (1965)).

Als wirksam werden diejenigen Dosen angesehen, die die Ausscheidung von Oozysten und/oder klinische Symptome der Coccidiose einschließlich der Mortalität vollständig oder in hohem Maße verhüten. In der folgenden Tabelle werden die wirksamen Dosen angegeben:

**Tabelle 1**

**Coccidiose bei Hühnern**

| Beispiel Nr. | Dosis ppm. | Sterberate tot/eingesetzt | Oocystenausscheidung in % im Vergleich zur unbehandelten infizierten Kontrolle | Gewichtszunahme in % im Vergleich zur nicht infizierten unbehandelten Kontrolle | Blutausscheidung mit dem Kot |
|---|---|---|---|---|---|
| unbehandelte infizierte Kontrolle | | 2/6 | 100 | 35 | stark |
| 4 | 50 | 0/3 | 0 | 100 | keine |
| | 25 | 0/3 | 0 | 100 | keine |
| | 10 | 0/6 | 0 | 100 | keine |

Herstellungsbeispiele

I Beispiele für Verfahren 2a

Beispiel 1

2-[4[(4′Chloro)-2′-thiazolyloxy]phenyl]-3,5(2H,4H)-dioxo-as-triazin

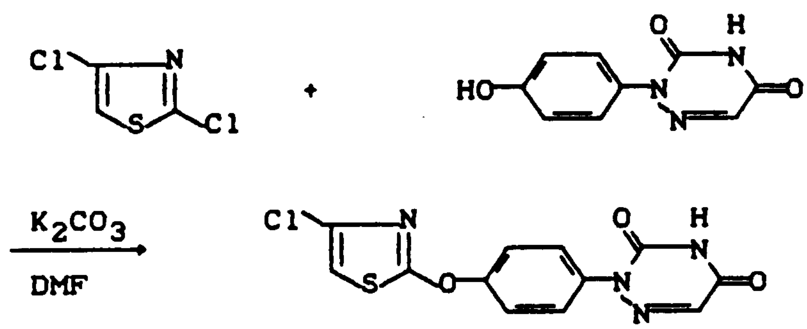


29 (0,01 mol) Hydroxyphenylazauracil, 1,5 g (0,01 mol) Dichlorthiazol und 1,4 g (0,01 mol) Kaliumcarbonat werden in 20 ml trockenem DMF unter Rückfluß 2h gerührt. Das abgekühlte Reaktionsgemisch wird mit HCl angesäuert und ausgefallenes Produkt abgesaugt. Nach dem Umkristallisieren aus Ethanol erhält man 2,9 g (90 % der Theorie) Thiazolyloxarylazaurazil.

Analog werden hergestellt

Beispiel 2

2-[-[4′-chloro-5′-methyl)-2′-thiazolyloxy]phenyl]1,2,4-triazin-3,5(2H,4H)dion.

Beispiel 3

2-(4-(2-benzthiazolyloxy)-phenyl)-1,2,4-triazin-3,5(2H,4H)dion.

Beispiel 4

2-[4[6′-Chloro)2′-benzthiazolyloxy]-3,5-dichlorophenyl]1,2,4-triazin-3,5(2H,4H)dion.

II Beispiel für Verfahren 2b

Beispiel 5

2-[4-[(4′-chloro)-2′-thiazolyloxy]phenyl]-3-N-methyl-3,5-(2H,4H)-dioxo-1,2,4-triazin

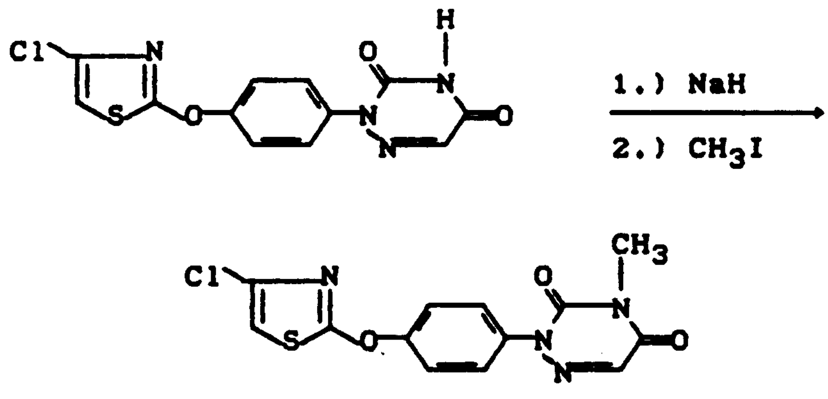

2 g (6 mmol) Thiazolyloxyarylazauracil werden in 20 ml absolutem DMSO gelöst und mit 0,14 g (6 mmol) Natriumhydrid versetzt. Man rührt 20 min bei RT und gibt dann 1,3 g (9 mmol) Methyljodid in 5 ml DMSO unter Argon zu. Man erwärmt auf 50 °C und hält 3h bei dieser Temperatur. Anschließend wird das Reaktionsgemisch i.V. eingeengt und dann mit Wasser versetzt. Nach dem Absaugen des ausgefallenen Feststoffs erhält man so 1,5 g (71 % der Theorie) der N-Methylverbindung.

Beispiele für Verfahren 2c)

2-[4-[6′-Chlor)2′-benzoxazolylsulfoxy]-3,5-dichlorophenyl]1,2,4-triazin-3,5(2H,4H)dion.

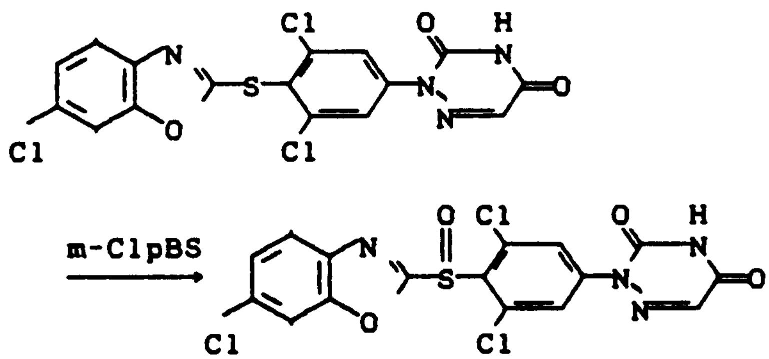


10 g (0,027 mol) Chlorbenzoxazolylthiophenylazauracil werden in einem Gemisch aus 200 ml Methanol und 100 ml Dichlormethan gelöst. Man kühlt auf 10 °C ab und gibt bei dieser Temperatur 4,6 g m-Chlorperbenzoesäure (85 %ig) zu. Nach 10 h Nachrühren bei 10 °C wird das Lösungsmittel im Vakuum abgezogen und der Rückstand aus Isopropanol umkristallisiert. Man erhält so 8,5 g (82 % der Theorie) Sulfoxid.

Analog werden hergestellt:

Beispiel 6

2-[4-(2'-Benzoxazolylsulfoxyl)-3,5-dichlorophenyl]1,2,4-triazin-3,5(2H,4H)dion.

Beispiel 7

2-[4-[(6'-Chloro)-2'-benzoxazolylsulfonyl]-3,5-dichlorophenyl)1,2,4-triazin-3,5(2H,4H)dion.

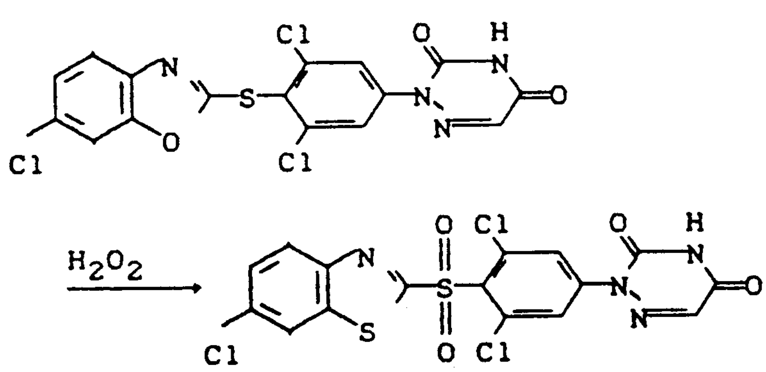


8,8 g (0,02 mol) Chlorbenzoxazolylthiophenylazauracil werden in 100 ml Eisessig gelöst und mit 40 ml 30 %igem Wasserstoffperoxid 18 h unter Rückfluß gerührt. Nach dem Abkühlen gibt man Wasser zu und saugt den ausgefallenen Niederschlag ab. Umkristallisieren aus Isopropanol liefert 6,9 g Sulfon (73 % der Theorie).

Analog werden hergestellt:

Beispiel 8

2-[4-(2'-Benzoxazolylsulfonyl)-3,5-dichlorophenyl]1,2,4-triazin-3,5(2H,4H)dion.

Beispiel 9

2-[4-(2'-Benzoxazolylsulfonyl)-phenyl]1,2,4-triazin-3,5(2H,4H)dion.

III Beispiel für Verfahren 4

IIIa)

2-(4-Hydroxyphenyl)-1,2,4-triazin-3,5(2,4H)dion

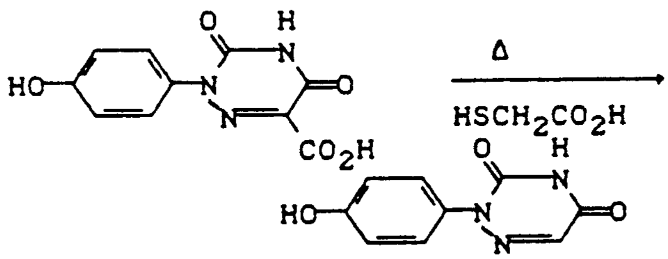


34 g (0,137 mol) Carbonsäure werden in 34 ml Mercaptoessigsäure auf 170 °C erhitzt. Nach 1,5 h läßt man abkühlen, versetzt mit Wasser und erhält nach Abfiltration 24 g (82 % der Theorie) decarboxyliertes Produkt.

Analog werden hergestellt.

IIIb)

2-(3,5-Dichlor-4-hydroxyphenyl)-1,2,4-triazin-3,5(2H,4H)dion

IIIc)

2-(3-Methyl-4-hydroxyphenyl)-1,2,4-triazin-3,5(2H,4H)dion.

IIId)

2-(4-Hydroxyphenyl)-3,5-(2H,4H)dion-1,2,4,-triazin

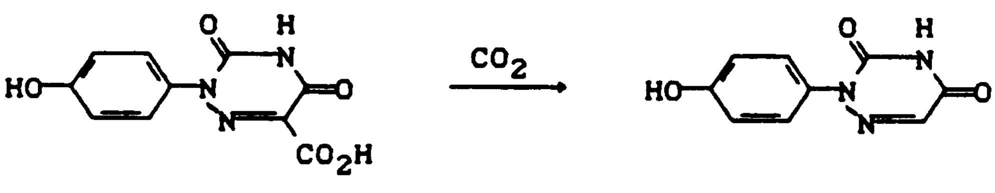


19 g (0,076 mol) Carbonsäure werden in einem Metallbad bei 260-280 °C unter Argon bis zur Beendigung der Gasentwicklung erhitzt. Nach dem Abkühlen wird der Rückstand aus Ethanol umkristallisiert. Es resultieren 10 g (64 % der Theorie) Azauracil.

IV Beispiele für Verfahren 6

IVa)

2-(4-Hydroxyphenyl)-3,5(2H,4H)dioxo-1,2,4,-triazin-6-carbonsäure

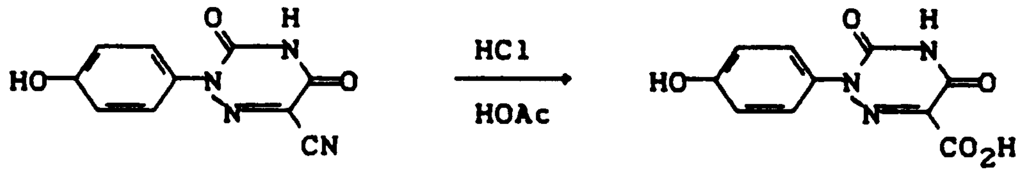


30,1 g (0,13 mol) Cyanazauracil werden 14 h in 1000 ml HCl/Eisessig (1:1) unter Rückfluß gerührt. Nach dem Abkühlen wird eingeengt und der Rückstand mit Wasser versetzt und ausgefallenes Produkt abgesaugt 19 g (59 % der Theorie).

Analog werden hergestellt

IVb)

2-(3,5-Dichlor-4-hydroxyphenyl)-1,2,4-triazin-3,5(2H,4H)dion-6-carbonsäure.

IVc)

-2(3-Methyl-4-hydroxyphenyl)-1,2,4-triazin-3,5(2H,4H)dion-6-carbonsäure.

V Beispiele für Verfahren 8

Va)

2-(4-Hydroxyphenyl)-3,5-(2H,4H),dioxo-6-cyano-1,2,4-triazin

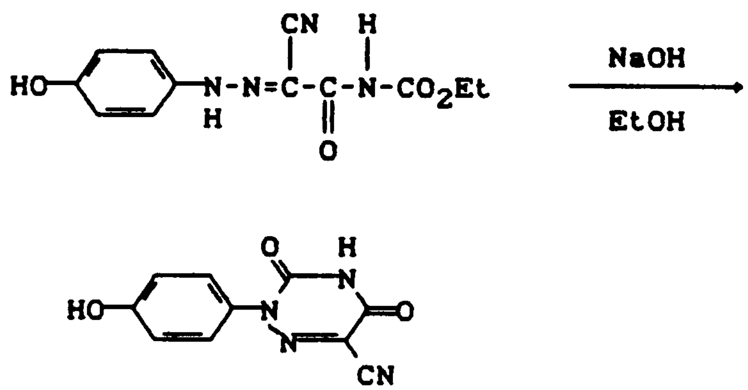


43,8 g (0,158 mol) des Hydrazonocyanurethans, 8,5 g (0,213 mol) NaOH werden in 400 ml abs. Ethanol 2 h unter Rückfluß erhitzt. Anschließend kühlt man ab, säuert mit Salzsäure an und engt i.V. ein. Man verrührt mit Wasser und saugt den ausgefallenen Niederschlag ab. Man erhält so nach Trocknung 30,1 g (85 % der Theorie) Cyanazauracil.

Vb)

2-(3,5-Dichlor-4-hydroxyphenyl)-6-cyano-1,2,4-triazin-3,5(2H,4H)dion

Vc)

2-(3-Methyl-4-hydroxyphenyl)-6-cyano-1,2,4-triazin.

VI Beispiele für Verfahren 10

VIa)

Ethyl-N-[[[Cyano(4-hydroxyphenyl)-hydrazinyliden]-methyl]carbonyl]-carbamt

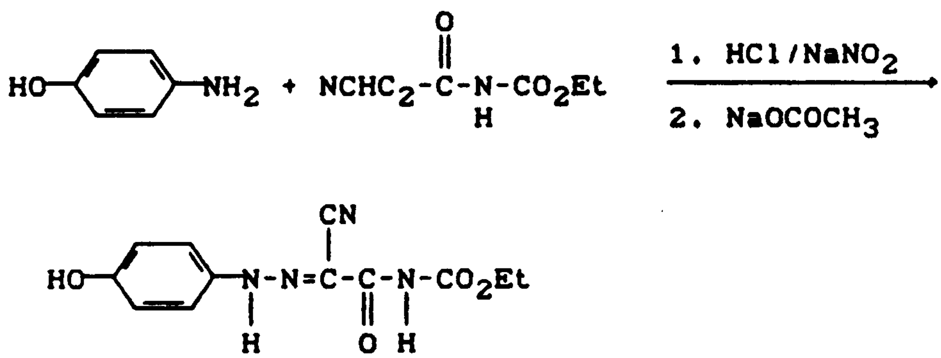


10 g (0,091 mol) 4-Hydroxyanilin werden in 19,7 ml konz. HCl und 200 ml Eisessig gelöst und bei 0-5 ° C mit einer Lösung aus 6,4 g (0,092 mol) Natriumnitrit in 30 ml Wasser tropfenweise versetzt. Man rührt bis zur klaren Lösung nach, gibt dann ein Gemisch aus 14,3 g (0,092 mol) Cyanacetylurethan und 21 g (0,25 mol) Natriumacetat zu und läßt 3 h bei 10 ° C nachrühren. Das Reaktionsgemisch wird i.V. eingeengt, mit Wasser verrührt und der Feststoff abgesaugt. Man erhält so 19 g (75 %) Produkt als feinkristallines gelbes Pulver.

Analog werden hergestellt

VIb)

Ethyl-N-[[[-Cyano(3,5-dichlor-4-hydroxyphenyl)-hydrazinyliden]methyl]carbonyl]-carbamat.

VIc)

Ethyl-N-[[[Cyano(3-methyl-4-hydroxyphenyl)-hydrazinyliden]methyl]carbonyl]-carbamat.

VId)

2-(3,5-Dichlor-4-hydroxyphenyl)-1,2,4-triazin-3,5(2,4H)dion

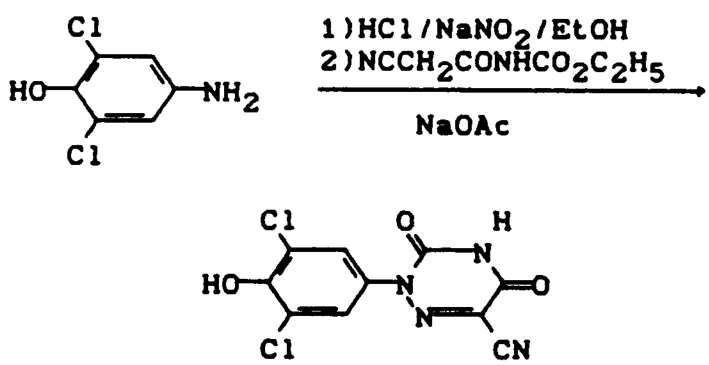

196 g (1,1 mol) 2,6-Dichlor-4-aminophenol werden in 2,4 l Ethanol und 240 ml konz. Salzsäure gelöst und auf 6 bis 10° gekühlt und bei dieser Temperatur langsam mit einer equimolaren wäßrigen Natriumnitrit-Lösung versetzt. Man läßt 30 Min. nachrühren und gibt dann nacheinander 400 g Natriumacetat und 172 g (1,1 mol) Cyanacetylurethan zu und rührt bei Raumtemperatur 1 Stunde nach. Anschließend erhitzt man 2 Stunden unter Rückfluß, kühlt ab und versetzt mit Wasser. Der ausgefallene Niederschlag wird abgesaugt und mit verd. HCl und Wasser gewaschen. Man erhält so 289 g (88 % der Theorie) Cyanazauracil.

**Patentansprüche**

**1.** Substituierte 1,2,4-Triazindione der allgemeinen Formel I

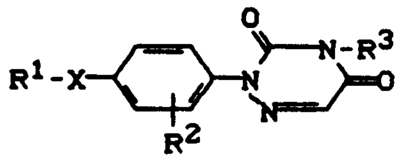

I

in welcher

$R^1$ für über Kohlenstoff gebundene heteroaromatische Reste steht, die gegebenenfalls substituiert sind,

X für O, S, SO, $SO_2$ steht,

$R^2$ für einen oder mehrere, gleiche oder verschiedene Reste der Gruppe Wasserstoff, Halogen, Nitro, Alkyl, Alkoxy, Alkylthio, Halogenalkyl, Halogenalkoxy und Halogenalkylthio,

$R^3$ für Wasserstoff, gegebenenfalls substituiertes Alkyl, Alkenyl, Alkinyl, Aralkyl steht.

**2.** Verfahren zur Herstellung substituierte 1,2,4-Triazindione der allgemeinen Formel I

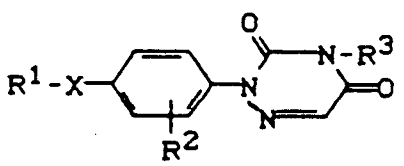

I

in welcher

$R^1$ für über Kohlenstoff gebundene heteroaromatische Reste steht, die gegebenenfalls substituiert sind,

X für O, S, SO, $SO_2$ steht,

$R^2$ für Wasserstoff einen oder mehrere, gleiche oder verschiedene Reste der Gruppe Wasserstoff, Nitro, Halogen, Alkyl, Alkoxy, Alkylthio, Halogenalkyl, Halogenalkoyy und Halogenalkylthio,

$R^3$ für Wasserstoff, gegebenenfalls substituiertes Alkyl, Alkenyl, Alkinyl, Aralkyl steht,

dadurch gekennzeichnet, daß man

a) Verbindungen der Formel II

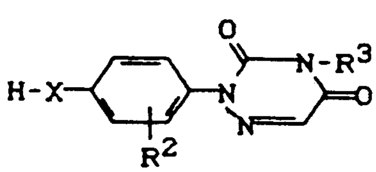

II

in welcher

X, $R^2$ $R^3$ die oben angegebene Bedeutung haben

mit Verbindungen der Formel III

$R^1$-A (III)

in welcher
$R^1$ die oben angegebene Bedeutung hat und
A für die Reste Halogen, -O-$SO_2$-Alkyl, -O-$SO_2$-Aryl, -O-$SO_2$-Halogenalkyl, -S-Alkyl , $SO_2$-Alkyl, $SO_2$-Halogenalkyl
umsetzt, oder
b) daß man zur Herstellung von Verbindungen der Formel I in welcher $R^3$ nicht für Wasserstoff steht, Verbindungen der Formel Ia

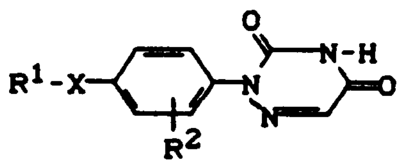

Ia

in welcher
$R^1$, $R^2$, X die oben angegebene Bedeutung haben mit Verbindungen der Formel IV

$R^3$-B (IV)

in welcher
$R^3$ für gegebenenfalls substituiertes Alkyl, Alkenyl, Alkinyl, Aralkyl steht und
B für Halogen, -O-$SO_2$-Alkyl, -O-$SO_2$-Halogenalkyl, -O-$SO_2$-Aryl steht
umsetzt, oder
c) daß man zur Herstellung von Verbindungen der Formel I, in welcher X für -SO- oder -$SO_2$-steht, Verbindungen der Formel I in welcher X für S steht mit einem Oxidationsmittel umsetzt.

**3.** Verbindungen der Formel II

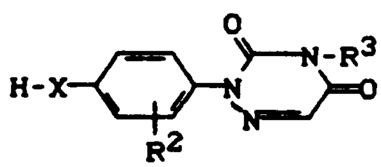

II

in welcher
X für O oder S steht,
$R^2$ für einen oder mehrere, gleiche oder verschiedene Reste an der Gruppe Halogen, Nitro, Alkyl, Alkoxy, Alkylthio, Halogenalkyl, Halogenalkoxy und Halogenalkylthio steht und für den Fall, daß X für S steht, zusätzlich für Wasserstoff steht,
$R^3$ für Wasserstoff, Alkyl, Alkenyl, Alkinyl, Aralkyl steht.

**4.** Verfahren zur Herstellung der neuen Verbindungen der Formel II gemäß Anspruch 3, dadurch gekennzeichnet, daß man Verbindungen der Formel V

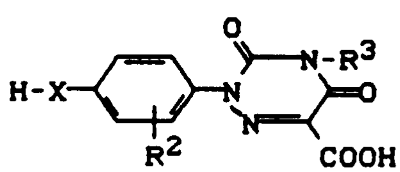

V

in welcher
X, $R^2$, $R^3$ die in Anspruch 3 angegebene Bedeutung haben,

durch Erhitzen decarboxyliert.

**5.** Verbindungen der Formel V

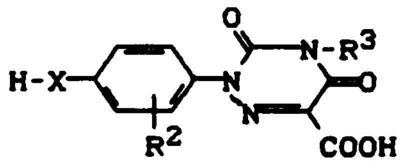

V

in welcher

X für O oder S steht,

$R^2$ für einen oder mehrere, gleiche oder verschiedene Reste aus der Gruppe Halogen, Nitro, Alkyl, Alkoxy, Halogenalkyl, Halogenalkoxy steht und für den Fall, daß X für S steht, zusätzlich für Wasserstoff steht,

$R^3$ für Wasserstoff, Alkyl, Alkenyl, Alkinyl, Aralkyl steht.

**6.** Verfahren zur Herstellung der neuen Verbindungen der Formel V gemäß Anspruch 5, dadurch gekennzeichnet, daß man Verbindungen der Formel VI

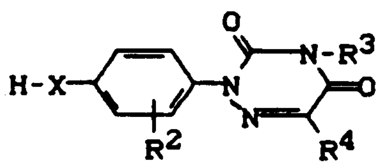

VI

in welcher

X, $R^2$, $R^3$ die in Anspruch 3 angegebene Bedeutung haben,

$R^4$ für die Reste -CN,

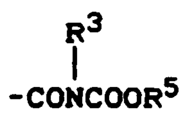

steht,

$R^5$ für gegebenenfalls substituiertes Alkyl oder Aryl steht

in Gegenwart anorganischer Säuren erhitzt.

**7.** Verbindungen der Formel VI

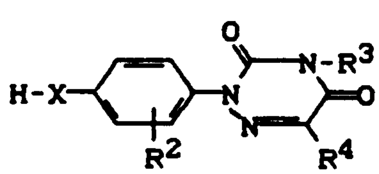

VI

in welcher

X, $R^2$, $R^3$, $R^4$ die in Anspruch 6 angegebene Bedeutung haben.

**8.** Verfahren zur Herstellung der neuen Verbindungen der Formel VI gemäß Anspruch 7 dadurch gekennzeichnet, daß man Verbindungen der Formel VII

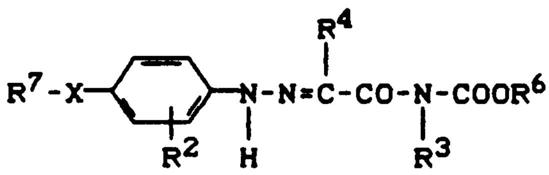 VII

in welcher

| | |
|---|---|
| X, $R^2$, $R^3$, $R^4$ | die in Anspruch 7 angegebene Bedeutung haben und |
| $R^6$ | für Alkyl oder gegebenenfalls substituiertes Aryl steht |
| $R^7$ | für Wasserstoff oder gegebenenfalls substituiertes |

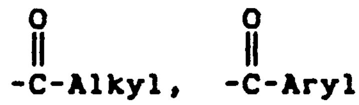

steht.

in Gegenwart von Basen erhitzt.

**9.** Verbindungen der Formel VII

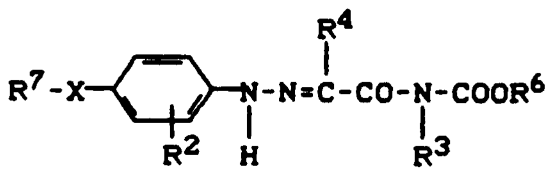 VII

in welcher

| | |
|---|---|
| X, $R^2$, $R^3$, $R^4$, $R^6$ und $R^7$ | die in Anspruch 8 angegebene Bedeutung haben und für den Fall, daß $R^7$ für H oder X für S steht, $R^2$ zusätzlich für Wasserstoff stehen kann. |

**10.** Verfahren zur Herstellung der neuen Verbindungen der Formel VII gemäß Anspruch 9 dadurch gekennzeichnet, daß man Verbindungen der Formel VIII

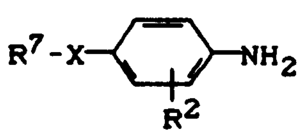 VIII

in welcher

| | |
|---|---|
| X, $R^2$ und $R^7$ | die in Anspruch 9 angegebene Bedeutung haben |

zunächst mit Alkalinitrit in Gegenwart von wäßrigen Mineralsäuren diazotiert und anschließend mit Verbindungen der Formel IX

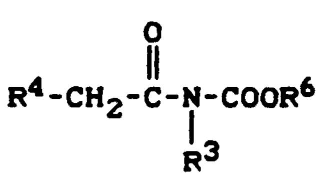

in welcher

| | |
|---|---|
| $R^3$, $R^4$ und $R^6$ | die in (9) angegebene Bedeutung besitzen, |

umsetzt.

**11.** Mittel gegen parasitische Protozoen, gekennzeichnet durch einen Gehalt an mindestens einem 1,2,4-Triazindion der Formel (I) gemäß Anspruch 1.

**12.** Verfahren zur Herstellung von Mitteln gegen parasitische Protozoen, dadurch gekennzeichnet, daß man 1,2,4-Triazindione der Formel (I) gemäß Anspruch 1 mit Streckmitteln und/oder oberflächenaktiven Mitteln vermischt.

**13.** Verwendung von 1,2,4-Triazindione der Formel (I) gemäß Anspruch 1 zur Herstellung von Mitteln gegen parasitische Protozoen.

**Claims**

**1.** Substituted 1,2,4-triazinediones of the general formula I

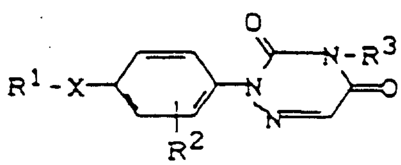

I

in which
- $R^1$ represents heteroaromatic radicals bonded via carbon, which are optionally substituted,
- X represents O, S, SO or $SO_2$,
- $R^2$ represents one or more, identical or different radicals from the group comprising hydrogen, halogen, nitro, alkyl, alkoxy, alkylthio, halogenoalkyl, halogenoalkoxy and halogenoalkylthio,
- $R^3$ represents hydrogen, optionally substituted alkyl, alkenyl, alkinyl or aralkyl.

**2.** Process for the preparation of substituted 1,2,4-triazinediones of the general formula I

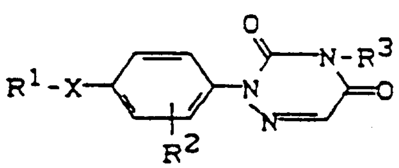

I

in which
- $R^1$ represents heteroaromatic radicals bonded via carbon, which are optionally substituted,
- X represents O, S, SO or $SO_2$,
- $R^2$ represents hydrogen, one or more, identical or different radicals from the group comprising hydrogen, nitro, halogen, alkyl, alkoxy, alkylthio, halogenoalkyl, halogenoalkoxy and halogenoalkylthio,
- $R^3$ represents hydrogen, optionally substituted alkyl, alkenyl, alkinyl or aralkyl,

characterized in that

a) compounds of the formula II

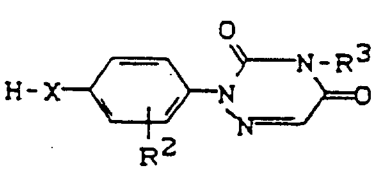

II

in which

X, $R^2$ and $R^3$ have the abovementioned meaning are reacted with compounds of the formula III

$R^1$-A (III)

in which

- $R^1$ has the abovementioned meaning and
- A represents the radicals halogen, O-$SO_2$-alkyl, -O-$SO_2$-aryl, -O-$SO_2$-halogenoalkyl, -S-alkyl, $SO_2$-alkyl and $SO_2$-halogenoalkyl,

or

b) in that for the preparation of compounds of the formula I in which $R^3$ does not represent hydrogen, compounds of the formula Ia

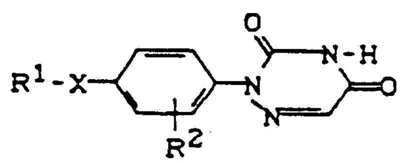

Ia

in which

$R^1$, $R^2$ and X have the abovementioned meaning, are reacted with compounds of the formula IV

$R^3$-B (IV)

in which

- $R^3$ represents optionally substituted alkyl, alkenyl, alkinyl or aralkyl and
- B represents halogen, -O-$SO_2$-alkyl, -O-$SO_2$-halogenoalkyl or -O-$SO_2$-aryl

or

c) in that for the preparation of compounds of the formula I in which X represents -SO- or -$SO_2$-, compounds of the formula I in which X represents S are reacted with an oxidant.

**3.** Compounds of the formula II

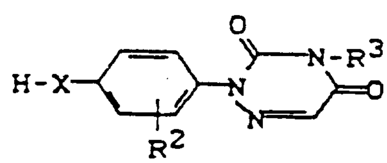

II

in which

- X represents O or S,
- $R^2$ represents one or more, identical or different radicals from the group comprising halogen, nitro, alkyl, alkoxy, alkylthio, halogenoalkyl, halogenoalkoxy and halogenoalkylthio and for the case in which X represents S, additionally represents hydrogen,
- $R^3$ represents hydrogen, alkyl, alkenyl, alkinyl or aralkyl.

**4.** Process for the preparation of the new compounds of the formula II according to Claim 3, characterized in that compounds of the formula V

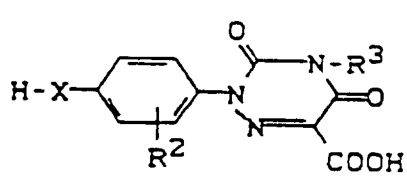

V

in which
X, $R^2$ and $R^3$ have the meaning indicated in Claim 3, are decarboxylated by heating.

**5.** Compounds of the formula V

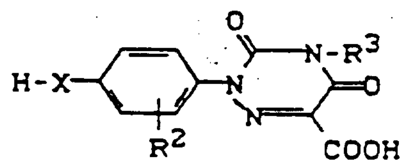

V

in which
X represents O or S,
$R^2$ represents one or more, identical or different radicals from the group comprising halogen, nitro, alkyl, alkoxy, halogenoalkyl and halogenoalkoxy and for the case in which X represents S, additionally represents hydrogen,
$R^3$ represents hydrogen, alkyl, alkenyl, alkinyl or aralkyl.

**6.** Process for the preparation of the compounds of the formula V according to Claim 5, characterized in that compounds of the formula VI

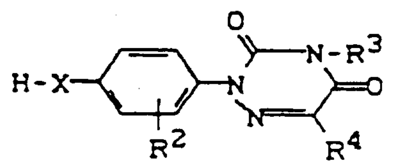

VI

in which
X, $R^2$ and $R^3$ have the meaning indicated in Claim 3,
$R^4$ represents the radicals -CN and

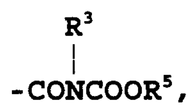

$R^5$ represents optionally substituted alkyl or aryl
are heated in the presence of inorganic acids.

**7.** Compounds of the formula VI

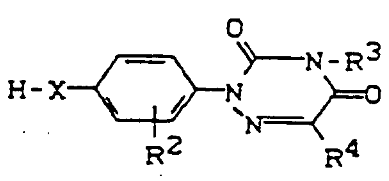

VI

in which
X, $R^2$, $R^3$ and $R^4$ have the meaning indicated in Claim 6.

**8.** Process for the preparation of the compounds of the formula VI according to Claim 7, characterized in that compounds of the formula VII

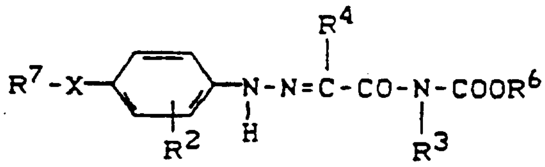

VII

in which

X, $R^2$, $R^3$ and $R^4$ have the meaning indicated in Claim 7,
$R^6$ represents alkyl or optionally substituted aryl and
$R^7$ represents hydrogen or optionally substituted

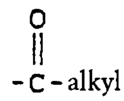 , 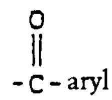

are heated in the presence of bases.

**9.** Compounds of the formula VII

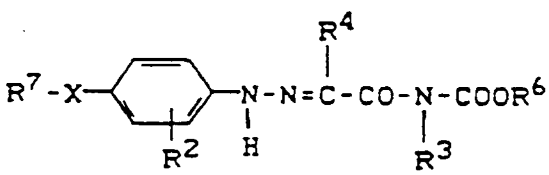

VII

in which

X, $R^2$, $R^3$, $R^4$, $R^6$ and $R^7$ have the meaning indicated in Claim 8 and for the case in which $R^7$ represents H or X represents S, $R^2$ can additionally represent hydrogen.

**10.** Process for the preparation of the compounds of the formula VII according to Claim 9, characterized in that compounds of the formula VIII

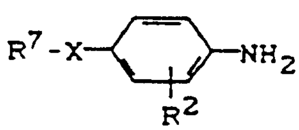

VIII

in which

X, $R^2$ and $R^7$ have the meaning indicated in Claim 9

are first diazotized using alkali metal nitrite in the presence of aqueous mineral acids and then reacted with compounds of the formula IX

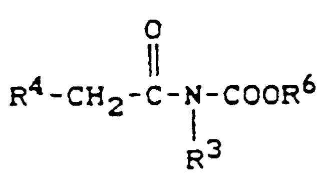

in which

$R^3$, $R^4$ and $R^6$ have the meaning indicated in Claim 9.

**11.** Agents against parasitic protozoa, characterized in that they contain at least one 1,2,4-triazinedione of the formula (I) according to Claim 1.

**12.** Process for the preparation of agents against parasitic protozoa, characterized in that 1,2,4-triazinediones of the formula (I) according to Claim 1 are mixed with extenders and/or surface-active agents.

**13.** Use of 1,2,4-triazinediones of the formula (I) according to Claim 1 for the preparation of agents against parasitic protozoa.

**Revendications**

**1.** 1,2,4-triazinediones substituées, de formule générale I

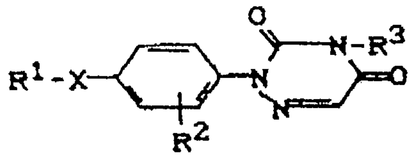

I

dans laquelle

$R^1$ représente des restes hétéroaromatiques fixés par l'intermédiaire du carbone et qui sont éventuellement substitués;

X représente O, S, SO, $SO_2$;

$R^2$ représente un ou plusieurs restes, identiques ou différents choisis dans l'ensemble formé par un atome d'hydrogène ou d'halogène, un reste nitro, alkyle, alcoxy, alkylthio, halogéno-alkyle, halogéno-alcoxy et halogéno-alkylthio;

$R^3$ représente un atome d'hydrogène ou un reste alkyle, alcényle, alcynyle, aralkyle éventuellement substitués.

**2.** Procédé de préparation de 1,2,4-triazinediones substituées de formule générale I

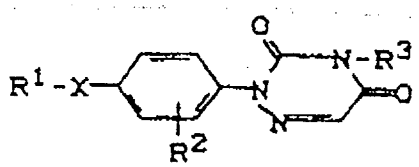

I

dans laquelle

$R^1$ représente des restes hétéroaromatiques fixés par l'intermédiaire du carbone qui sont éventuellement substitués,

X représente O, S, SO, $SO_2$;

$R^2$ représente un atome d'hydrogène ou un ou plusieurs restes identiques ou différents, choisis dans l'ensemble formé par un atome d'hydrogène, un reste nitro, halogéno, alkyle, alcoxy, alkylthio, halogéno-alkyle, halogéno-alcoxy et halogéno-alkylthio;

$R^3$ représente un atome d'hydrogène, un groupe alkyle, alcényle, alcynyle, aralkyle, éventuellement substitués

procédé caractérisé en ce que :

a) on fait réagir des composés de formule II

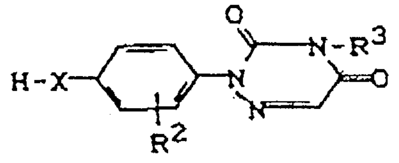

II

dans laquelle

X, $R^2$, $R^3$ ont le sens indiqué ci-dessus,

avec des composés de formule III

$R^1$-A (III)

dans laquelle

- $R^1$ a le sens indiqué ci-dessus, et
- A représente les restes halogène, -O-$SO_2$-alkyle, -O-$SO_2$-aryle -O-$SO_2$-halogéno-alkyle, -S-alkyle, -$SO_2$-alkyle, -$SO_2$-halogéno-alkyle,

ou

b) pour préparer des composés de formule I, dans laquelle $R^3$ ne représente pas un atome d'hydrogène, on fait réagir des composés de formule Ia.

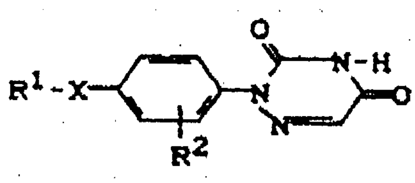

Ia

dans laquelle

- $R^1$, $R^2$, X ont le sens précité,

avec des composés de formule IV

$R^3$-B (IV)

dans laquelle

- $R^3$ représente un reste alkyle, alcényle, alcynyle, aralkyle, éventuellement substitués, et
- B représente un atome d'halogène, un reste -O-$SO_2$-alkyle, -O-$SO_2$-halogéno-alkyle, -O-$SO_2$-aryle,

ou bien

c) pour préparer des composés de formule I, dans laquelle X représente -SO- ou -$SO_2$-, on fait réagir des composés de formule I, dans laquelle X représente S, avec un oxydant.

**3.** Composés de formule II

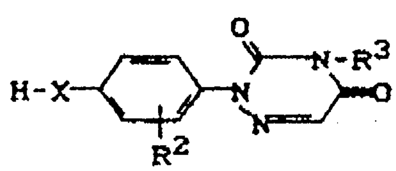

II

dans laquelle

- X représente O ou S,
- $R^2$ représente un ou plusieurs restes, identiques ou différents, choisis dans l'ensemble formé par un atome d'halogène, un reste nitro, alkyle, alcoxy, alkylthio, halogéno-alkyle, halogéno-alcoxy et halogéno-alkylthio, et, si X représente S, $R^2$ peut représenter en outre un atome d'hydrogène,
- $R^3$ représente un atome d'hydrogène, un reste alkyle, alcényle, alcynyle, aralkyle.

**4.** Procédé de préparation des nouveaux composés de formule II selon la revendication 3, caractérisé en ce qu'on soumet des composés de formule V

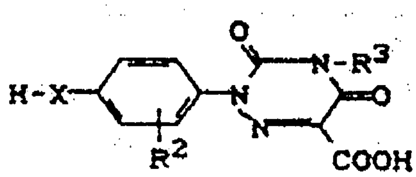

V

dans laquelle

X, $R^2$ et $R^3$ ont le sens indiqué à la revendication 3, à une décarboxylation par chauffage

**5.** Composés de formule V

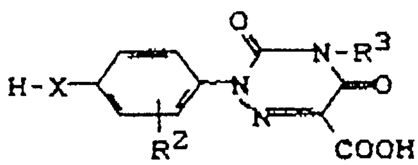

V

dans laquelle

- X représente O ou S,
- $R^2$ représente un ou plusieurs restes identiques ou différents choisis dans l'ensemble formé par un atome d'halogène, un reste nitro, alkyle, alcoxy, halogénoalkyle, halogéno-alcoxy, et halogénoalkylthio, et, si X représente S, $R^2$ peut représenter en outre un atome d'hydrogène,
- $R^3$ représente un atome d'hydrogène, un reste alkyle, alcényle, alcynyle, aralkyle.

**6.** Procédé pour produire les nouveaux composés de formule V selon la revendication 5, caractérisé en ce qu'on chauffe en présence d'acides minéraux, des composés de formule VI :

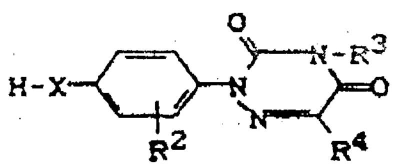

VI

dans laquelle

- X, $R^2$, $R^3$ ont le sens indiqué à la revendication 3,
- $R^4$ représente les restes -CN,

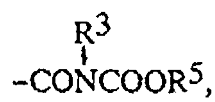

- $R^5$ représente un reste alkyle ou aryle, éventuellement substitués.

**7.** Composés de formule VI :

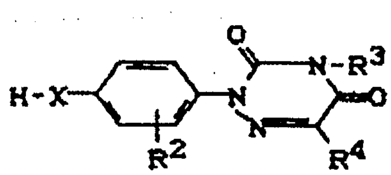

VI

dans laquelle

X, $R^2$, $R^3$, $R^4$ ont le sens indiqué à la revendication 6.

**8.** Procédé pour préparer les nouveaux composés de formule VI selon la revendication 7, caractérisé en ce qu'on chauffe en présence de bases des composés de formule VII :

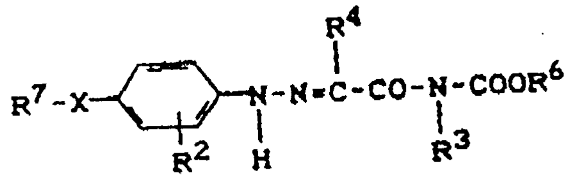

VII

dans laquelle

X, $R^2$, $R^3$, $R^4$ ont le sens indiqué à la revendication 7, et

$R^6$ représente un reste alkyle ou un reste aryle éventuellement substitués,

$R^7$ représente un atome d'hydrogène ou un reste

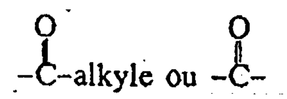

aryle éventuellement substitués.

**9.** Composés de formule VII

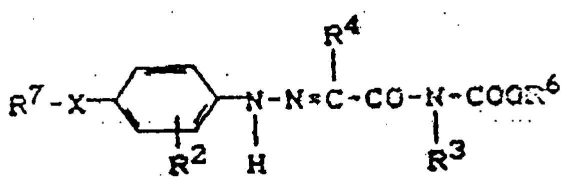

VII

dans laquelle

X, $R^2$, $R^3$ $R^4$, $R^6$ et $R^7$ ont le sens indiqué à la revendication 8 et, si

$R^7$ représente H ou si X représente S, $R^2$ peut représenter en outre un atome d'hydrogène.

**10.** Procédé de préparation des nouveaux composés de formule VII selon la revendication 9, caractérisé en ce qu'on soumet des composés de formule VIII :

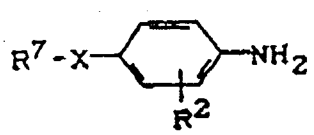

VIII

dans laquelle

X et $R^2$ et $R^7$ ont le sens indiqué en (9),

tout d'abord à une diazotation avec un nitrite alcalin en présence d'acides minéraux aqueux puis à une réaction avec des composés de formule IX :

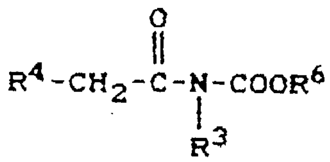

IX

dans laquelle

$R^3$, $R^4$ et $R^6$ ont le sens indiqué à la revendication 9.

**11.** Produit pour combattre des protozoaires parasitaires, caractérisé en ce que ce produit contient au moins une 1,2,4,-triazinedione de formule (I) selon la revendication 1.

**12.** Procédé pour préparer des produits pour combattre des protozoaires parasitaires, caractérisé en ce qu'on mélange des 1,2,4-triazinediones de formule (I) selon la revendication 1 avec des excipients, charges ou agents d'allongement et/ou avec des produits tensioactifs.

**13.** Utilisation de 1,2,4-triazinediones de formule (I) selon la revendication 1 pour préparer des produits pour combattre des protozoaires parasitaires.